# EUROPÄISCHE PATENTSCHRIFT

(11) **EP 2 209 463 B1**
(45) Veröffentlichungstag und Bekanntmachung des Hinweises auf die Patenterteilung: **05.04.2017**
(21) Anmeldenummer: 08802219.9
(22) Anmeldetag: 15.09.2008
(51) Int. Cl.: A61L 27/38, A61L 27/60, C12N 5/071, A61K 8/98, A61Q 19/04

(54) **VERFAHREN ZUM ERHÖHEN DER PIGMENTIERUNG VON HAUT UNTER VERWENDUNG VON MELANOZYTENVORLÄUFERZELLEN**
METHOD FOR INCREASING THE PIGMENTATION OF THE SKIN USING MELANOCYTE PRECURSOR CELLS
PROCÉDÉ D'AUGMENTATION DE LA PIGMENTATION DE LA PEAU PAR UTILISATION DE PRÉCURSEURS DE MÉLANOCYTES

(30) Priorität: 15.10.2007 DE 102007049402
(43) Veröffentlichungstag der Anmeldung: 28.07.2010
(73) Patentinhaber: Sinclair Pharmaceuticals Limited, Chester CH4 9QZ (GB)
(72) Erfinder: HUNZIKER, Thomas, CH-3653 Oberhofen (CH)
(74) Vertreter: Bobbert & Partner Patentanwälte PartmbB
(86) Internationale Anmeldenummer: PCT/EP2008/007684
(87) Internationale Veröffentlichungsnummer: WO 2009/049734

(56) Entgegenhaltungen:
- WO-A-01/40444
- WO-A-2006/005977
- MA H ET AL: "A new method for purifying amelanotic melanocytes from human hair follicles" JOURNAL OF INVESTIGATIVE DERMATOLOGY, Bd. 122, Nr. 3, März 2004 (2004-03), Seite A103, XP002544109 & 65TH ANNUAL MEETING OF THE SOCIETY-FOR-INVESTIGATIVE-DERMATOLOGY; PROVIDENCE, RI, USA; APRIL 28 -MAY 01, 2004 ISSN: 0022-202X
- NA G Y ET AL: "Isolation and characterization of outer root sheath melanocytes of human hair follicles." THE BRITISH JOURNAL OF DERMATOLOGY NOV 2006, Bd. 155, Nr. 5, November 2006 (2006-11), Seiten 902-909, XP002544110 ISSN: 0007-0963
- TOBIN DESMOND J ET AL: "Different populations of melanocytes are present in hair follicles and epidermis" PIGMENT CELL RESEARCH, Bd. 9, Nr. 6, 1996, Seiten 304-310, XP002544111 ISSN: 0893-5785
- LIMAT ALAIN ET AL: "USE OF EPIDERMAL EQUIVALENTS GENERATED FROM FOLLICULAR OUTER ROOT SHEATH CELLS IN VITRO AND FOR AUTOLOGOUS GRAFTING OF CHRONIC WOUNDS" CELLS TISSUES ORGANS, KARGER, BASEL, CH, Bd. 172, Nr. 2, 1. Januar 2002 (2002-01-01), Seiten 79-85, XP008074938 ISSN: 1422-6405
- VANSCHEIDT WOLFGANG ET AL: "Repigmentation by Outer-Root-Sheath-Derived Melanocytes: Proof of Concept in Vitiligo and Leucoderma" DERMATOLOGY (BASEL), Bd. 218, Nr. 4, 2009, Seiten 342-343, XP009122165 ISSN: 1018-8665

## Beschreibung

Die vorliegende Erfindung betrifft eine Melanozytenvorläuferzellen-Suspension gemäß dem Oberbegriff des Anspruchs 1.

Aus der Praxis sind Personen bekannt, deren Haut an einer oder mehreren Stellen eine Minderpigmentierung gemessen an anderen Hautarealen aufweisen. Diese Minderpigmentierung kann mehr oder weniger blass gegenüber regulär pigmentierter Haut wirken und wird von manchen der Betroffenen als ästhetisch bzw. kosmetisch störend empfunden. Im Zeitalter des Faceliftings, der Verwendung von Botox zur Verbergung von Falten und dem Absaugen von Fett aus kosmetischen Gründen besteht daher der Wunsch, auch eine Erhöhung bzw. eine Verstärkung der Pigmentierung bestimmter Hautbereiche bewirken zu können.

Die WO 2006/005977 A1 beschreibt ein Verfahren zur Kultivierung von Melanozyten zur späteren Verwendung in Hauttransplantaten.

MA H ET AL: "A new method for purifying amelanotic melanocytes from human hair follicles" JOURNAL OF INVESTIGATIVE DERMATOLOGY, Bd. 122, Nr. 3, März 2004 (2004-03), Seite A103, XP002544109 & 65TH ANNUAL MEETING OF THE SOCIETY-FOR-INVESTIGATIVE-DERMATOLOGY; PROVIDENCE, RI, USA; APRIL 28 - MAY 01, 2004 ISSN: 0022-202X, beschreiben ein Verfahren zum Aufreinigen von Hautwurzelscheidenzellen.

Na et al., "Isolation and characterization of outer root sheath melanocytes of human hair follicles", British Journal of Dermatology 2006, 155, Seite 902-909, XP002544110 ISSN: 0007-0963, beschreiben ein Verfahren zur Kultivierung von Melanozyten aus der äußeren Haarwurzelscheide.

TOBIN DESMOND J ET AL: "Different populations of melanocytes are present in hair follicles and epidermis" PIGMENT CELL RESEARCH, Bd. 9, Nr. 6, 1996, Seiten 304-310, XP002544111 ISSN: 0893-5785 beschreiben Arten von Melanozyten der äußeren Haarwurzelscheide.

LIMAT ALAIN ET AL: "USE OF EPIDERMAL EQUIVALENTS GENERATED FROM FOLLICULAR OUTER ROOT SHEATH CELLS IN VITRO AND FOR AUTOLOGOUS GRAFTING OF CHRONIC WOUNDS" CELLS TISSUES ORGANS, KARGER, BASEL, CH, Bd. 172, Nr. 2, 1. Januar 2002 (2002-01-01), Seiten 79-85, XP008074938 ISSN: 1422-6405, diskutieren die Verwendungen von Zellen der äußeren Haarwurzelscheide.

Die WO 01/40444 A (BIOTISSUE TECHNOLOGIES AG [DE]) 7. Juni 2001 (2001-06-07) befasst sich mit Zellen des menschlichen Körpers.

VANSCHEIDT WOLFGANG ET AL: "Repigmentation by Outer-Root-Sheath-Derived Melanocytes: Proof of Concept in Vitiligo and Leucoderma" DERMATOLOGY (BASEL), Bd. 218, Nr. 4, 2009, Seiten 342-343, XP009122165 ISSN: 1018-8665, befassen sich mit der der Repigmentierung von Haut.

Erfindungsgemäße Aufgabe ist es daher, eine geeignete Melanozytenvorläuferzellen-Suspension zum Erhöhen bzw. Verstärken der Pigmentierung von Haut sowie Applikationsmodalitäten derselben anzugeben. Die erfindungsgemäße Aufgabe wird gelöst durch eine Melanozytenvorläuferzellen-Suspension mit den Merkmalen des Anspruchs 1.

Das Aufbringen von Melanozytenvorläuferzellen bzw. -stammzellen aus Haarwurzelscheiden, insbesondere aus epithelialen Haarwurzelscheiden, eines ersten Hautbereichs auf einen zweiten Hautbereich dient der Erhöhung der Pigmentierung des zweiten Hautbereichs durch Pigmentbildung am zweiten Hautbereich durch Ausbildung insbesondere von Melanozyten aus den Vorläuferzellen mit deren bekanntem Beitrag zur Pigmentierung der Haut. Die Pigmentierung ist dabei den äußeren Umständen - wie bspw. der Stärke oder der Dauer einer Sonneneinstrahlung - entsprechend veränderbar und passt sich somit kosmetisch vorteilhaft auf natürliche Weise der Pigmentierung des den zweiten Hautbereich umgebenden Hautareals an.

Das Aufbringen von Melanozytenvorläuferzellen bringt im Gegensatz zum Aufbringen von bspw. interfollikulären Melanozyten ferner den Vorteil einer vergleichsweise unbegrenzten Verfügbarkeit mit sich. Während die Gewinnung von Melanozyten i.d.R. mit einer Entnahme von Haut einhergeht und damit nur zur Erhöhung der Pigmentierung eines begrenzten Hautareals in Frage kommt - und zudem mit einem chirurgischen Eingriff in die Integrität der Haut an der Entnahmestelle einhergeht - können Melanozytenvorläuferzellen vergleichsweise einfach und in hoher Stückzahl aus Haarwurzelscheiden gewonnen werden. Das Aufbringen von Melanozytenvorläuferzellen impliziert somit deren vorteilhaft hohe Verfügbarkeit.

Das Aufbringen kann bspw. mittels einer Suspension mit 10²-10⁹, insbesondere mit 10⁵-10⁷ Zellen/ml, bspw. mittels Spritze oder Spray oder in einem biokompatiblen Vlies erfolgen.

Dies kann in einer biokompatiblen Lösung (z. B. PBS, Fibrin) oder mittels eines biokompatiblen Trägers (z. B. Hyaluronan, Kollagen) erfolgen. Die Zellen können dabei als vitale oder wachstumsarretierte (z. B. mittels Mitomycin C oder Bestrahlung wachstumsarretierte) Zellen oder auch als Zellextrakte (wie z. B. Lyophilisate, Sonicate) eingesetzt werden. Auch von diesen Zellen konditionierte Medien können zu diesem Zweck eingesetzt werden.

Das Aufbringen von Melanozytenvorläuferzellen impliziert ferner deren vorteilhaft einfache und somit wiederholt durchführbare Gewinnung. So kann der Vorgang des Gewinnens dieser Melanozytenvorläuferzellen vor ihrer Aufbringung vergleichsweise einfach und schmerzfrei durchgeführt werden. Dieser Vorgang birgt ferner kein Komplikationsrisiko. So können die zur Zellgewinnung verwendeten Haare bspw. durch ein Auszupfen von Kopfhaaren, insbesondere von Anagenhaaren, insbesondere vom Capillitium gewonnen sein, was einen weiteren Vorteil gegenüber der Verwendung von Zellen anderer Herkunft, insbesondere von interfollikulären Melanozyten bedeutet.

Die Melanoytenvorläuferzellen können dabei von einem Spender stammen und bei diesem auch wieder aufgebracht werden. Der erste Hautbereich und der zweite Hautbereich können somit Hautbereiche ein und desselben Lebewesens sein. Die Melanoytenvorläuferzellen können jedoch auch von einem Spender stammen und bei einem von diesem verschiedenen Empfänger aufgebracht werden. Dabei ist es unerheblich, ob Spender und Empfänger Mensch oder Tier sind. Auch eine Übertragung von Tier auf Mensch und umgekehrt ist von der Erfindung umfasst. Unter "Melanozytenvorläuferzellen" werden erfindungsgemäß sowohl autologe als auch allogene und xenogene Melanozytenvorläuferzellen bzw. -stammzellen verstanden.

Das Aufbringen der Vorläuferzellen kann hierbei durch einfaches Auftragen auf die Haut erfolgen. Dabei können die Zellen mittels z. B. Fibrinkleber auf dem zweiten Hautbereich fixiert und mit einem Okklusionsverband geschützt werden. Aber auch jede andere geeignete Form des Aufbringens z. B. durch Integration der Zellen in biologische oder synthetische Matrices ist erfindungsgemäß möglich.

Vorteilhafte Weiterbildungen der vorliegenden Erfindung sind dabei jeweils Gegenstand der Unteransprüche.

So weist die erfindungsgemäße Melanozytenvorläuferzellen-Suspension in einer bevorzugten Ausführungsform als zusätzlichen Schritt ihrer Herstellung das Gewinnen der Melanozytenvorläuferzellen auf.

Gewinnen im Sinne des Patents bedeutet zunächst das Trennen der Melanozytenvorläuferzellen vom ersten Hautbereich. Es kann erfindungsgemäß auch ein Lösen der Melanozytenvorläuferzellen vom ersten Hautbereich umfassen. Dieses Lösen kann bspw. ein einfaches Auszupfen von Haaren, insbesondere Anagen-Haaren aus der Kopfhaut, umfassen.

Dabei können die auf der Haut des Empfängers aufgebrachten Melanozytenvorläuferzellen insbesondere auch von diesem Empfänger selber stammen. Spender und Empfänger können somit identisch sein. Ist dies der Fall, so entfallen - oder verringern sich - vorteilhaft Bemühungen, welche ein Typisieren oder Matchen aufgrund von genetischen Unterschieden zwischen Spender und Empfänger betreffen. Zudem entfallen Risiken der Übertragung - bspw. von Infektionen - vom Spender auf den Empfänger.

Zum "Gewinnen" von Melanozytenvorläuferzellen aus Haarwurzelscheiden kann erfindungsgemäß neben dem Trennen der Melanozytenvorläuferzellen vom ersten Bereich - oder alternativ hierzu - auch das Isolieren der Melanozytenvorläuferzellen aus den Haarwurzelscheiden, insbesondere aus epithelialen Haarwurzelscheiden, zählen.

Zum Schritt des "Gewinnens" kann auch ein Schritt oder eine Mehrzahl von Schritten zählen, mittels welcher die gewonnenen Zellen zu ihrem Aufbringen vorbereitet werden. Dies kann mittels Herstellen einer Melanozytenvorläuferzellen-Suspension direkt, d. h. nicht nach in vitro Vermehrung geschehen. Diese Suspension kann neben den Melanozytenvorläuferzellen biokompatible Substanzen wie PBS, und/ oder Fibrin und/ oder einen biokompatiblen Träger wie bspw. Hyaluronan oder Kollagen umfassen.

In einer weiter bevorzugten erfindungsgemäßen Ausführungsform wird vorgeschlagen, dass zusätzlich Keratinozytenvorläuferzellen aufgebracht werden. Dies kann gleichzeitig mit dem Schritt des Aufbringens der Melanozytenvorläuferzellen erfolgen. Die Keratinozyten- und die Melanozytenvorläuferzellen können jedoch auch zeitlich versetzt aufgebracht werden. In einer weiter bevorzugten Ausführungsform gilt das oben zum Gewinnen der Melanozytenvorläuferzellen Gesagte ebenso für die Keratinozytenvorläuferzellen. Auch das oben zur Herkunft der Zellen (Tier, Mensch, autogen, autolog, usw.) Gesagte trifft auf die Kerotinozytenvorläuferzellen zu.

Ein mittels der erfindungsgemäßen Melanozytenvorläuferzellen-Suspension dieser beiden zuletzt genannten Ausführungsformen jeweils erzielbarer Vorteil liegt darin, dass die gemeinsame und ggf. auch gleichzeitige Verwendung von Melanozytenvorläuferzellen und Keratinozytenvorläuferzellen zu einem begünstigten Wachstum nach ihrem gemeinsamen Aufbringen auf den zweiten Hautbereich führt. Die Erfinder der vorliegenden Melanozytenvorläuferzellen-Suspension führen dies auf Interaktionen chemischer, biochemischer und/ oder biologischer Art zwischen den genannten Vorläuferzellen-Typen zurück. Sie konnten beobachten, dass dieser Vorteil bereits dann auftritt, wenn das natürliche Mischungsverhältnis von Keratinozytenvorläuferzellen zu Melanozytenvorläuferzellen, wie es bspw. in den Haarwurzelscheiden des ersten Hautbereichs vorliegt, auch in der auf den zweiten Hautbereich aufgebrachten Mischung dieser Zellen beibehalten wird.

In einer wiederum weiter bevorzugten erfindungsgemäßen Ausführungsform wird der zweite Hautbereich zur Aufnahme der Melanozytenvorläuferzellen - und ggf. auch der Keratinozytenvorläuferzellen - vorbereitet. Diese Vorbereitung erlaubt besonders wirksam ein Anwachsen der aufgebrachten Vorläuferzellen auf dem zweiten Hautbereich.

Das Vorbereiten des zweiten Hautbereichs kann bspw. ein Entfernen der Epidermis, oder Teilen hiervon, des zweiten Hautbereichs umfassen. Letzteres ist bspw. mittels Dermabrasio oder oberflächlicher Laserapplikation mit den hiermit verbundenen, dem Fachmann bekannten Vorteilen möglich.

Zum Vorbereiten kann auch das Aufbringen einer geeigneten Lösung zählen. Als Beispiel sei hier eine Fibrin-Lösung genannt, welche den zweiten Hautbereich zur späteren Aufnahme der Vorläuferzellen und zu deren besseren Anhaften und vor allem Anwachsen am zweiten Hautbereich vorbereitet.

In einer wiederum weiter bevorzugten erfindungsgemäßen Ausführungsform werden die auf den zweiten Hautbereich aufgebrachten Vorläuferzellen zum beschleunigten Aufbau von Pigmenten stimuliert.

Eine solche Stimulierung kann mittels UV-Belichtung erfolgen. Diese aktiviert die transferierten Melanozytenvorläuferzellen und kann z. B. mittels Breitband-UV, Schmalband-UVB, PUVA oder Excimer-Laser-Bestrahlung erfolgen.

Dieses Stimulieren führt vorteilhaft zu einer Pigmentierung sowie dem Aufbau der epidermalen Pigmenteinheit. Eine Stimulierung bspw. mittels Ultraviolett-Belichtung kann einmalig oder wiederholt erfolgen. Die Bestrahlung sollte dabei vorteilhaft jeweils unterhalb der Erythem-Schwelle liegen. Eine zweimalige Bestrahlung pro Woche bis zur Erzielung der gewünschten Pigmentierung des zweiten Hautbereichs hat sich dabei als wirkungsvoll erwiesen. Eine häufigere oder weniger häufige Bestrahlung ist ebenfalls möglich.

Erfindungsgemäß ist es vorgesehen, die Vorläuferzellen - seien es die Melanozytenvorläuferzellen oder die Keratinozytenvorläuferzellen - enzymatisch von der Haarwurzelscheide eines entnommenen Haars zu lösen. Die enzymatische Ablösung kann bspw. mittels einer Trypsin/EDTA-Lösung erfolgen. Diese Lösung kann 0,8 %-ig vorliegen und Zimmertemperatur, bspw. 20 °C, aufweisen. Die Lösung bewirkt insbesondere eine Ablösung der epithelialen Zellen vom Haarschaft.

Zur enzymatischen Ablösung kann eine Inkubation in Trypsin, 0,1 % bis 10 %, besonders zwischen 0,5 % bis 4 %, in PBS über 10 - 50, insbesondere über 15 - 30 min, erfolgen.

Vorteilhaft an diesem Vorgehen ist insbesondere, dass durch die mögliche Verwendung von Enzymen eine nachteilige Auswirkung auf etwa die behandelten Vorläuferzellen vermieden werden kann. Die enzymatische Ablösung ist daher besonders schonend für die zu gewinnenden Zellen.

Weitere mögliche Schritte hiervon umfassen das Stoppen des enzymatischen Ablösens durch die Zugabe von Humanserum, das Zentrifugieren der Suspension zum Erhalten eines Sediments sowie das erneute Suspendieren des zellhaltigen Sediments in einer Thrombin-Lösung, welche eine sofortige Fixierung der aufgebrachten Zellen in dünner Schicht bei der Applikation auf vorangehend aufgebrachtes Fibrinogen erlaubt und somit eine homogene, nicht zu okklusive Applikation in jeder Körperregion ermöglicht.

Die Erfindung umfasst in weiteren bevorzugten Ausführungsformen - jeweils unabhängig voneinander - die Schritte: Überführen der Vorläuferzellen oder der Suspension oder des Sediments in eine biokompatible Lösung, Einbringen der Vorläuferzellen oder der Suspension oder des Sediments in einen biokompatiblen Träger und/ oder Herstellen eines Zellextraktes.

Im Folgenden wird ein exemplarisches Ausführungsbeispiel detailliert beschrieben.

So wurden bei einer Person mit einem seit Jahren unverändert bestehenden Vitiligoherd von rund zehn Quadratzentimetern Fläche am Handrücken 50 Anagenhaare aus der Kopfhaut gezupft. Die abgetrennten Haarwurzeln wurden für 25 min in Trypsin/EDTA-Lösung, 0,8 %-ig, bei Zimmertemperatur inkubiert. Hierbei lösten sich epitheliale Zellen vom Haarschaft. Die Reaktion wurde durch Zugabe von Humanserum beendet. Die mit der Trypanblau-Exlusion gemessene Vitalität der Zellen lag dabei bei über 50 %. Die Zellsuspension wurde bei 500 g zentrifugiert, und das zellhaltige Sediment wurde in 2 ml einer Thrombin-Lösung suspendiert. Die zur Erhöhung der Pigmentierung vorgesehene Fläche am Handrücken wurde nach eingehender Desinfektion mittels Dermabrasio deepidermisiert. Nach Aufbringen von 2 ml einer Fibrin-Lösung auf die Wundfläche wurde die Zellsuspension aufgetragen. Das Behandlungsareal wurde anschließend mit einem herkömmlichen Wundverband okklusiv abgedeckt. Ein Verbandswechsel erfolgte alle drei Tage. Nach einer innerhalb von zwei Wochen abgeschlossenen Reepithelisierung wurde das Behandlungsareal zwei Mal wöchentlich mit Breitspektrum-Ultraviolett unterhalb der Erythem-Schwelle belichtet. Eine vollständige Angleichung der Pigmentierung des behandelten Hautbereichs an eine diesen umgebende Haut konnte auf diese Weise im Verlauf von acht Wochen erzielt werden.

Zum Unterdrücken einer Autoimmunreaktion können im Falle einer Vitiligo zeitlich begrenzt geeignete Wirkstoffe wie topische oder systemische Kortikosteroide oder topische Calcineurin-Inhibitoren eingesetzt werden.

## Patentansprüche

1. Melanoyztenvorläuferzellen-Suspension zur Anwendung in einem Verfahren zum Erhöhen der Pigmentierung von Haut, bei welchem Melanozytenvorläuferzellen aus Haarwurzelscheiden, welche von einem ersten Hautbereich eines Spenders gewonnen wurden, auf einen zweiten Hautbereich eines Empfängers aufgebracht werden,
wobei die Melanozytenvorläuferzellen enzymatisch von der Haarwurzelscheide abgelöst wurden,
wobei die Melanoyztenvorläuferzellen in die Melanoyztenvorläuferzellen-Suspension eingebracht wurden,
wobei die Melanoyztenvorläuferzellen direkt, nicht nach in vitro Vermehrung in die Melanoyztenvorläuferzellen-Suspension eingebracht wurden,
**dadurch gekennzeichnet, dass**
die Melanoyztenvorläuferzellen-Suspension direkt, nicht nach in vitro Vermehrung der Melanoyztenvorläuferzellen auf den zweiten Hautbereich aufgebracht wird.

2. Melanoyztenvorläuferzellen-Suspension zur Anwendung nach Anspruch 1, wobei der Spender der Empfänger ist.

3. Melanoyztenvorläuferzellen-Suspension zur Anwendung nach einem der vorangegangenen
Ansprüche, wobei die Melanoyztenvorläuferzellen-Suspension Keratinozytenvorläuferzellen umfasst.

4. Melanoyztenvorläuferzellen-Suspension Zur Anwendung nach einem der vorangegangenen
Ansprüche, wobei das enzymatischen Ablösen durch die Zugabe von Humanserum gestoppt wurde.

5. Melanoyztenvorläuferzellen-Suspension zur Anwendung nach einem der vorangegangenen
Ansprüche, wobei zum Erhalten eines zellhaltigen Sediments eine Suspension zentrifugiert wurde.

6. Melanoyztenvorläuferzellen-Suspension zur Anwendung nach Anspruch 5, wobei das zellhaltige Sediment in einer Thrombin-Lösung suspendiert wurde.

7. Melanoyztenvorläuferzellen-Suspension zur Anwendung nach einem der Ansprüche 5 bis 6,
wobei die Suspension oder das Sediment in eine biokompatible Lösung übergeführt wurde.

8. Melanoyztenvorläuferzellen-Suspension zur Anwendung nach einem der Ansprüche 5 bis 6,
wobei die Suspension oder das Sediment in einen biokompatiblen Träger eingebracht wurde.

## Claims

1. Melanocyte precursor cells suspension for use in a method for increasing the pigmentation of skin, wherein the melanocyte precursor cells from root sheaths, which have been obtained from a first area of skin of a donor, are applied to a second area of skin of a recipient,
wherein the melanocyte precursor cells have been enzymatically detached from the root sheaths,
wherein the melanocyte precursor cells have been introduced into the melanocyte precursor cells suspension,
wherein the melanocyte precursor cells have been directly introduced into the melanocyte precursor cells suspension, and not after in vitro cell cytokinesis,
**characterized in that**
the melanocyte precursor cells suspension is directly applied to the second area of skin, and not after in vitro cytokinesis of the melanocyte precursor cells.

2. Melanocyte precursor cells suspension for use according to claim 1, wherein the donor is the recipient.

3. Melanocyte precursor cells suspension for use according to any one of the preceding claims, wherein the melanocyte precursor cells suspension comprises keratinocyte precursor cells.

4. Melanocyte precursor cells suspension for use according to any one of the preceding claims, wherein the enzymatic detachment has been stopped by adding human serum.

5. Melanocyte precursor cells suspension for use according to any one of the preceding claims, wherein a suspension has been centrifuged to obtain a cell-containing sediment.

6. Melanocyte precursor cells suspension for use according to claim 5, wherein the cell-containing sediment has been suspended in a thrombin solution.

7. Melanocyte precursor cells suspension for use according to any one of claims 5 to 6, wherein the suspension or the sediment has been transferred into a biocompatible solution.

8. Melanocyte precursor cells suspension for use according to any one of claims 5 to 6, wherein the suspension or the sediment has been included into a biocompatible carrier.

## Revendications

1. Suspension de cellules précurseurs de mélanocytes pour utilisation dans un procédé d'augmentation de la pigmentation de la peau, dans lequel des cellules précurseurs de mélanocytes de bulbes pilaires obtenus d'une première surface de peau d'un donneur sont appliqués sur une seconde surface de peau d'un receveur,
où les cellules précurseurs de mélanocytes ont été séparées des bulbes pilaires de façon enzymatique,
où les cellules précurseurs de mélanocytes ont été introduites dans la suspension de cellules précurseurs de mélanocytes,
où les cellules précurseurs de mélanocytes ont été introduites directement dans la suspension de cellules précurseurs de mélanocytes, et non pas après multiplication in vitro,
**caractérisée en ce que**
la suspension de cellules précurseurs de mélanocytes est appliquée directement sur seconde surface de peau, et non pas après multiplication in vitro des cellules précurseurs de mélanocytes.

2. Suspension de cellules précurseurs de mélanocytes pour utilisation selon la première revendication, où le donneur est le receveur.

3. Suspension de cellules précurseurs de mélanocytes pour utilisation selon l'une quelconque des revendications précédentes, où la suspension de cellules précurseurs de mélanocytes comprend des cellules précurseurs de kératinocytes.

4. Suspension de cellules précurseurs de mélanocytes pour utilisation selon l'une quelconque des revendications précédentes, où la séparation enzymatique a été interrompue par addition de sérum humain.

5. Suspension de cellules précurseurs de mélanocytes pour utilisation selon l'une quelconque des revendications précédentes, où une suspension a été centrifugée dans le but d'obtenir un sédiment cellulaire.

6. Suspension de cellules précurseurs de mélanocytes pour utilisation selon la revendication 5, où le sédiment cellulaire a été mis en suspension dans une solution de thrombine.

7. Suspension de cellules précurseurs de mélanocytes pour utilisation selon l'une quelconque des revendications 5 ou 6, où la suspension, ou le sédiment, a été transféré(e) dans une solution biocompatible.

8. Suspension de cellules précurseurs de mélanocytes pour utilisation selon l'une quelconque des revendications 5 ou 6, où la suspension , ou le sédiment, a été transféré (e) dans un support biocompatible.
